# EUROPEAN PATENT APPLICATION

(11) **EP 3 910 342 A1**
(43) Date of publication of application: **17.11.2021**
(21) Application number: 20174693.0
(22) Date of filing: 14.05.2020
(51) Int. Cl.: G01N 35/00, A61B 10/00, A61B 10/02

(54) **A ROBOTIC SAMPLING APPARATUS AND A METHOD FOR OBTAINING AN INTRA-CAVITY BIOLOGICAL SURFACE SAMPLE FROM A PATIENT**

(71) Applicant: Syddansk Universitet, 5230 Odense M (DK)
(72) Inventor: Savarimuthu, Thiusius, Rajeeth, 5250 Odense SV (DK); Petersen, Henrik, Gordon, 5210 Odense SV (DK); Iversen, Nicolai, 5000 Odense C (DK); Kramberger, Aljaz, 5220 Odense SØ (DK); San Juan, Iñigo, Iturrate, 5000 Odense C (DK); Wilm, Jakob, 5000 Odense C (DK); Schmidt, Michael, Kjær, 5240 Odense NØ (DK); Denisa, Miha, 5230 Odense M (DK); Lindvig, Anders, Prier, 5000 Odense C (DK); Buch, Anders, Glent, 5240 Odense NØ (DK); Winther, Trine, Straarup, 5000 Odense C (DK); Sloth, Christoffer, 5000 Odense C (DK)
(74) Representative: Plougmann Vingtoft a/s

(57) **Abstract**

The present invention concerns a robotic sampling apparatus for obtaining a biological sample from a patient, comprising a preparation compartment comprising a plurality of storage areas accommodating individual test kit objects and a transfer arrangement for accommodating and repositioning at least one test kit object, and a first handling system for collecting at least one test kit object from at least one of said storage areas and positioning said at least one test kit object in said transfer arrangement; a sanitation compartment, which is adapted to receiving the at least one test kit object on the transfer arrangement, and wherein said sanitation compartment comprises a second handling system for picking a test kit object for obtaining the biological sample from the patient, an opening for a patient area for providing the patient in a predetermined position which is accessible to the second handling system for moving said test kit object from an initial position to an active position in which the biological sample can be obtained from the patient; and a control system provided for governing at least the first handling system, the movement of the transfer arrangement and the second handling system for performing of the robotic sampling apparatus when obtaining the biological sample from a patient.

## Description

### FIELD OF THE INVENTION

The present invention relates to an apparatus and a method for obtaining an intra-cavity biological surface sample from a patient.

### BACKGROUND OF THE INVENTION

The spread of virus through the global populous is recurrent in history, the most resent being the coronavirus which emerged in the Asian continent in late 2019 where it was identified in early 2020 and was later named COVID-19. The virus spread rapidly to other areas of the word in the following period. The COVID-19 outbreak was soon thereafter declared for a pandemic.

Previously in history, the world has witnessed other virus based pandemics, such as the "Spanish flu" pandemic of 1918, the avian Influenza A H5N1 virus ("Bird flu") epidemic of 2004, and the H 1N 1 viral strain ("Swine flu") pandemic of 2009. These pandemics cause global problems, both economically and medically. Besides these virus-based pandemics, influenza virus epidemics are frequently reoccurring.

In order to determine if a human being is infected with a virus, such as for instance the COVID-19, heath care staff perform a throat swab for collecting a sample swab from the throat. The aim of the swabbing procedure is to collect a high number of virus particles from the patient's throat. There is evidence that sampling should occur from the posterior parts of the oral cavity and the posterior nasopharyngeal wall of the mouth of the patient.

The inhalation of air contaminated by harmful virus and/or other micro-organisms is a common route for infection of human beings, particularly health care staff and others caused to work with infected humans or animals. Air exhaled by infected patients is a source of contamination. Masks are used as a barrier to prevent species-to-species transmission of the virus. However, especially health care staff taking throat samples from patients are particularly exposed and although they are wearing masks and similar barriers, there is a risk of cross contamination between heath care staff and patients in the sampling taking operation.

In order to control a pandemic outbreak a vast amount of swab tests must be taken. This increases the contamination risk, but is reduced by using protective clothes, masks, protective eyewear and the like. This however is costly as all of these protective measures are for single use only or must be sterilised between each use.

### OBJECT OF THE INVENTION

In order to reduce the risk of cross contamination the idea has arisen of creating an automated system for collecting a sample swab from a patient. Accordingly, an object for the present invention is to provide an automatic biologic sample swab on humans.

### SUMMARY OF THE INVENTION

This object is achieved by a robotic sampling apparatus for obtaining a biological sample from a patient, comprising
- a preparation compartment comprising
   a plurality of storage areas accommodating individual test kit objects and a transfer arrangement for accommodating and repositioning at least one test kit object, and
   a first handling system for collecting at least one test kit object from at least one of said storage areas and positioning said at least one test kit object in said transfer arrangement;
- a sanitation compartment, which is adapted to receiving the at least one test kit object on the transfer arrangement, and wherein said sanitation compartment comprises
   a second handling system for picking a test kit object for obtaining the biological sample from the patient,
   an opening for a patient area for providing the patient in a predetermined position which is accessible to the second handling system for moving said test kit object from an initial position to an active position in which the biological sample can be obtained from the patient, and
- a control system provided for governing at least the first handling system, the movement of the transfer arrangement and the second handling system for performing of the robotic sampling apparatus when obtaining the biological sample from a patient.

Hereby, an automated apparatus for taking biological samples is provided which is eliminating or at least substantially reducing the risk of contamination of the people involved in the operation, i.e. both the patient and the health care staff.

Advantageously, the preparation compartment may be environmentally controlled and a sterile environment is kept therein. This eliminates any risk of contaminating the test kit objects before the objects are used for taking a biological sample.

In one embodiment of the invention, the test kit objects include a sample container with a lid, a sample probe tool with a sample probe and a carriage adapted for accommodating a container with a lid and a probe tool. The tools may preferably be adapted to being operated by the handling systems. The sample containers used today normally come with a lid which is removed by unscrewing. However, it may be envisaged that other types of sample containers may be used in the robotic sampling apparatus according to the invention.

In a preferred embodiment, the storage areas include exchangeable cartridges for the test kit objects.

Preferably, the preparation compartment further comprises a labelling device for printing a personalised label based on identification data from the patient and that the label is applied to the container on the transfer arrangement. Additionally, the patient area further comprises a data input device, such as a card reader, for receiving identification data from the patient and obtaining label data to the labelling device, said label data may comprise the identification data and/or database data associated with said identification data. Hereby, the individual samples are marked and unambiguously related to the individual patients.

In order to maintain sterility, the preparation compartment and the sanitation compartment are divided by an isolated seal, such as a dividing wall with an automated gate therein. In addition, the transfer arrangement may be adapted to moving the at least one test kit object thereon from the preparation compartment to the sanitation compartment through the automated gate.

Preferably, the transfer arrangement is provided as a conveyor belt on which the at least one test kit object is placed on in the preparation compartment and then transferred to the sanitation compartment.

In the preferred embodiment of the invention, the sanitation compartment comprises an outlet for receiving the obtained sample herein for further processing, and a disposal bin for collecting objects other than the sample probe used for obtaining the biological sample. Hereby, the sample container as well as the other test kit objects used for obtaining the sample can be disposed of by being handled by the robot in the sanitation compartment.

In an embodiment, the sanitation compartment comprises a pneumatically actuated clamp for fixing a container for screwing on the lid by the second handling system. Hereby, the standard test kit containers with screw lids can be automatically handled by a robot. However, by the invention it is realised that other container designs may be used.

In the preferred embodiment of the invention, one or more sterilisation systems are provided for decontamination of at least the sanitation compartment and the transfer arrangement. The sterilisation systems may include ultraviolet radiation, application of disinfectants or a combination thereof. Hereby, cross contamination between patients can be avoided.

In the currently preferred embodiment of the invention, the biological sample from the patient is a throat swab sample and the predetermined position of the patient in the patient area comprises a head support for providing the mouth of the patient in an accessible manner to the second handling system. However, it is realised that the sampling apparatus according to the invention may be designed for obtaining an intra-cavity biological surface sample other than a mouth or throat swab from a patient.

Advantageously, the first handling system is a robot and/or the second handling system is a robot, such as a motorized multi-degree of freedom motion system.

According to a second aspect of the invention there is provided a method of obtaining a biological sample from a patient, said method comprising - preparing a sample probe for testing in a preparation compartment comprising a plurality of storage areas accommodating individual test kit objects and a transfer arrangement, and a first handling system for collecting at least one test kit object from at least one of said storage areas and positioning said at least one test kit object in said transfer arrangement;
- receiving the at least one test kit object on the transport device in a sanitation compartment, and wherein said sanitation compartment comprises a second handling system, an opening for a patient area for providing the patient in a predetermined position which is accessible to the second handling system, and
- moving said test kit object with the second handling system from an initial position to an active position where the biological sample is obtained from the patient by performing a swab,
- dispatching the obtained sample in an outlet for further processing, and collecting objects other than the sample probe used for obtaining the biological sample in a disposal bin; and
- controlling in a control system at least the first handling system, the movement of the transfer arrangement and the second handling system for operating a robotic sampling apparatus for obtaining the biological sample from a patient.

### BRIEF DESCRIPTION OF THE DRAWINGS

In the following, the invention is described in more detail with reference to the accompanying figures, in which
Figure 1 is a schematic perspective view of a robotic sampling apparatus according to an embodiment of the invention;
Figure 2 is an open top view of the robotic sampling apparatus;
Figure 3 is a cross-sectional side view of same;
Figure 4 is a perspective detailed view of the robotic sampling apparatus;
Figure 5 is a work flow diagram for a robotic sampling apparatus according to an embodiment of the invention;
Figure 6 is a process diagram for the preparation compartment of a robotic sampling apparatus according to one embodiment of the invention; and
Figure 7 is a process diagram for the sanitation compartment of a robotic sampling apparatus according to one embodiment of the invention;

### DETAILED DESCRIPTION

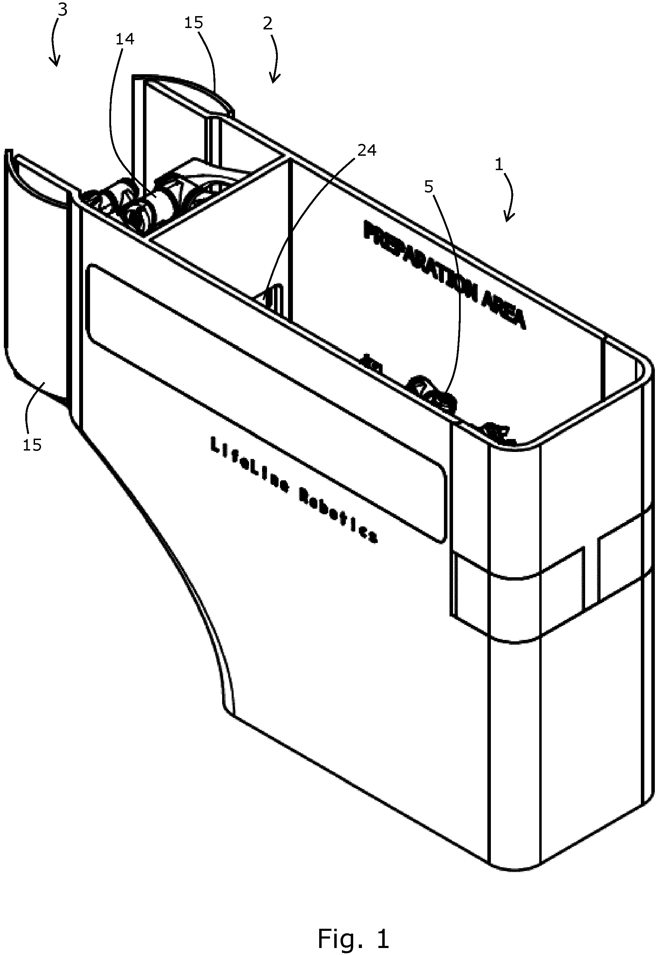

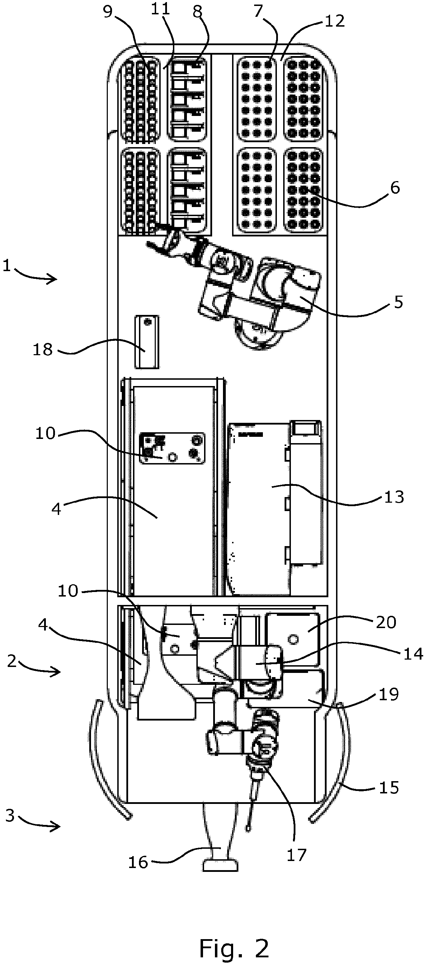

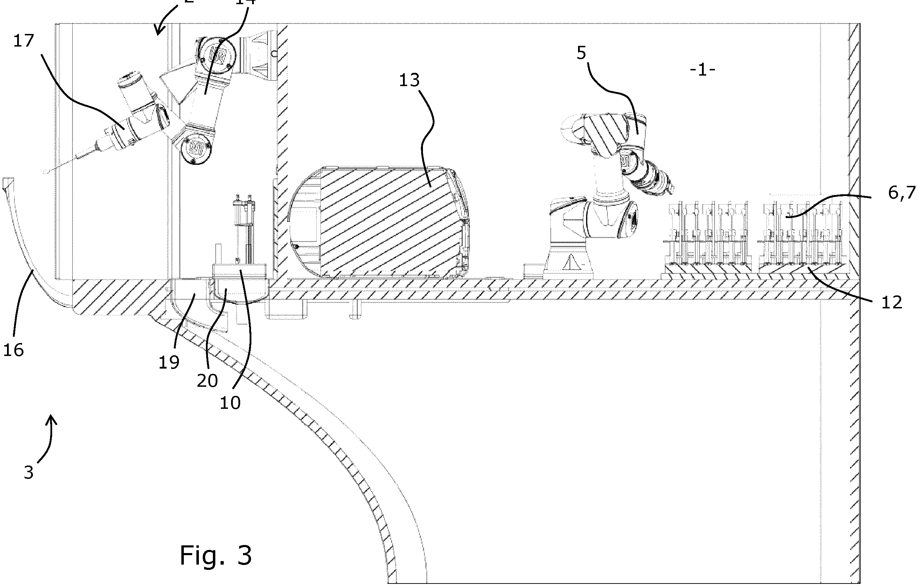

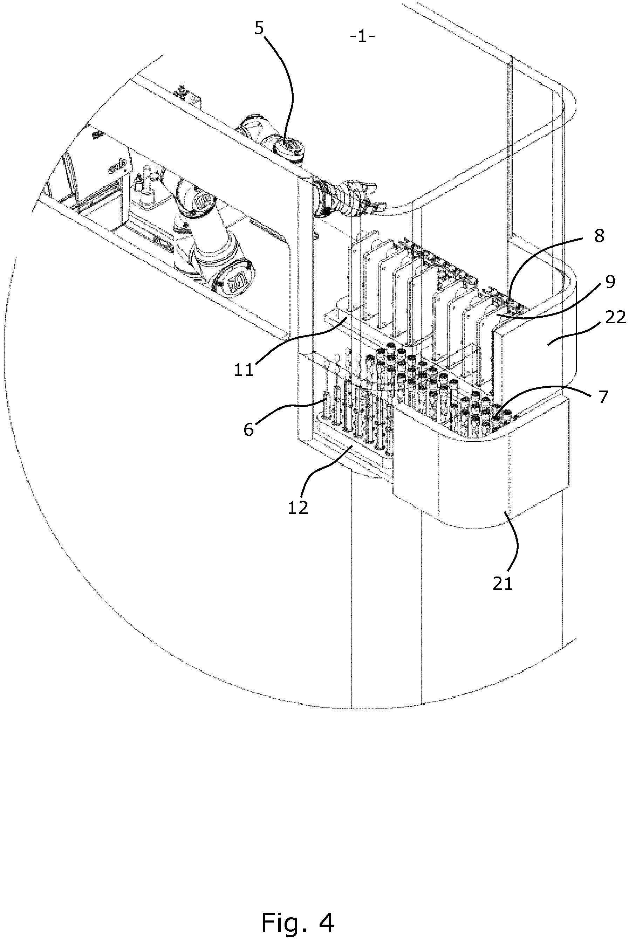

With reference to figures 1 to 4, an embodiment of the robotic sampling apparatus according to the invention is shown. The robotic sampling apparatus is shown without top cover. The sampling apparatus is divided into three sections: Preparation compartment 1, Sanitation compartment 2 and the Patient area 3. The preparation compartment 1 is kept as a sterile environment. In this compartment 1 the preparation of the necessary equipment, such as e.g. tools and one or more test kit objects 6, 7, 8, 9, is executed with no risk of contamination. The required test kit objects 6-9 are taken from their storage areas 11, 12, which may be exchangeable cartridges, by a handling system 5, preferably a robot, and collected on a carriage 10 or the like. After the test kit objects 6-9 are prepared on a transfer arrangement 4, for instance in the form of a slider or a conveyor belt, the carriage 10 are transported to the sanitation area 2, which has a status of a medium risk contamination area. The handling of the equipment 6-9 is done with tools designed for single use only, meaning they are required to be sanitized after each patient. In the sanitation compartment 2, a second handling robot 14 is provided, and once the robot 14 in the sanitation area 2 enters the sample acquisition phase, the patient doors 15 open and the robot 14 operates in an environment considered as high contamination risk area for the shortest amount of time possible. When the sample is obtained the doors close and the robot finishes the handling of the sample for future examination. The returning conveyor belt 4 progresses through a sanitation station, such as a three-step sanitation station comprising spraying of alcohol or other disinfectant, drying and applying a UV light before entering back into the preparation area. Meanwhile the sanitation compartment 2 goes through a similar three-step process after each patient.

The packaging facilitating the probe and the probe container, used for the automatic testing is optimized for human usage. Therefore, it is especially hard to manipulate and extract the probe and the container with the robot. For this reason, a fixture 11, 12 may be provided, comprised of two hand operated clamps and clamping strips, for clamping the packaging. This way the packaging was fixed tightly enough so that the robot could grasp and peel away the packaging.

For testing purposes the probe and the probe container with the accompanying tools are sterile, placed in fixtures or cartridges 11, 12 and loaded into the preparation compartment 1 by a human operator.

A carriage 10 serves as a movable fixture between the two compartments 1, 2. The carriages 10 are not one-time use parts but can be sanitized after every use. After sanitation, they are loaded in fixtures 11, 12 in the preparation compartment 1, where they can be picked and placed on the transfer arrangement 4 by the robot 5.

The carriage 10 is designed in such a way that all of the tools 6-9 can easily be accessible by the secondary robot 14 in the sanitation compartment 2. The tools and parts 6-9 are constrained with mechanical geometrical features, which are designed in such a way that the parts can still be easily placed and picked from the carriage 10.

A labelling device 13 is also provided in the preparation compartment 1. The robot operation of labelling involves that the probe containers with the attached lids are at first loaded into the preparation compartment 1 in fixtures 11, 12. The fixture constrains the movement of the container so that it can be picked from a predefined position with the two-finger gripper installed on the robot 5. After this action the robot 5 moves to the labelling machine 13 and applies the label. For a successful application, a specialized label application support add-on may be mounted on the printer. The add-on may be a flexible support for supporting and maintaining the contact pressure between the label and the container during the application process.

The next step is to unscrew the container lid. Therefore, the container has to be fixed permanently in order the robot can execute the unscrewing action. For this reason, a pneumatically actuated clamp 18 may be used. The clamp 18 may comprise a pneumatic cylinder, movable clamping piece, which is attached to the cylinder and enhanced with rubber fillets on the container contact surfaces to increase friction and a rigid base. The base is designed in a way that it can facilitate all of the calm components and it can be attached to the table. After the unscrewing of the lid is executed, the cylinder is released and the container can be picked a placed in the designated fixture on the carriage. In the apparatus two clamps 18 are installed, one in the preparation compartment 1 and one (not shown) in the sanitation compartment 2 for clamping the container during the lid screwing on action.

A further tool is the container tool. The main function of the tool is to manipulate the container from the carriage to the container clamp and later dispose it into the sanitation bin 19.

The purpose of isolating the preparation compartment 1 from the sanitation compartment 2 is minimizing the risk of contaminating the workspace.

The compartments 1, 2 are divided by a fixed wall, which facilitates an automated gate 24 to ensure an isolated seal between the two environments when engaged. The gate 24 can only be operated when the sanitation compartment 2 has gone through a three-step sanitation procedure and when the patient area doors 15 are properly closed.

In the current configuration of the sampling apparatus, the parts necessary for obtaining a sample includes: carriage, probe tool, container tool, lid tool, sample container and lid. To feed these test kit objects 6-9 to the sanitation compartment 2 a transfer arrangement 4 is integrated in the apparatus. The carriage 10 was designed to fix the tools in pre-defined positions to ensure positional repeatability for the robots 5, 14 when handling the test kit objects 6-9.

The tools are intended to be one-time-use and after finishing their task they are placed in the sanitation bin 19 for future sanitation before re-entering the sampling apparatus. The carriage 10 is also placed for future sanitation after a successful sampling cycle. As in the preparation compartment 1, the sanitation compartment 2 has a pneumatically actuated clamp for fixing the container when the robot 14 screws on the lid at the final stage of the cycle. The sample can then be placed in its final destination bin 20.

After delivering the final sample, and ensuring that the gate 24 and doors 15 are closed, the robot 14, moves to a predefined position while sanitation alcohol is sprayed through nozzles (not shown) to the robot 14 and the sanitation compartment 2 cleaning the entire compartment 2. Afterwards the compartment 2 is dried by blowing air from the top down, ensuring all the alcohol drops are collected at the bottom of the sanitation compartment. Lastly UV light is turned for a short time, ensuring that the compartment 2 is properly sanitized. All equipment used for the sample acquisition is considered contaminated and stored in a sealed container (not shown). When full the tools are extracted and sanitized in an autoclave before being loaded into the apparatus again in the preparation compartment 1.

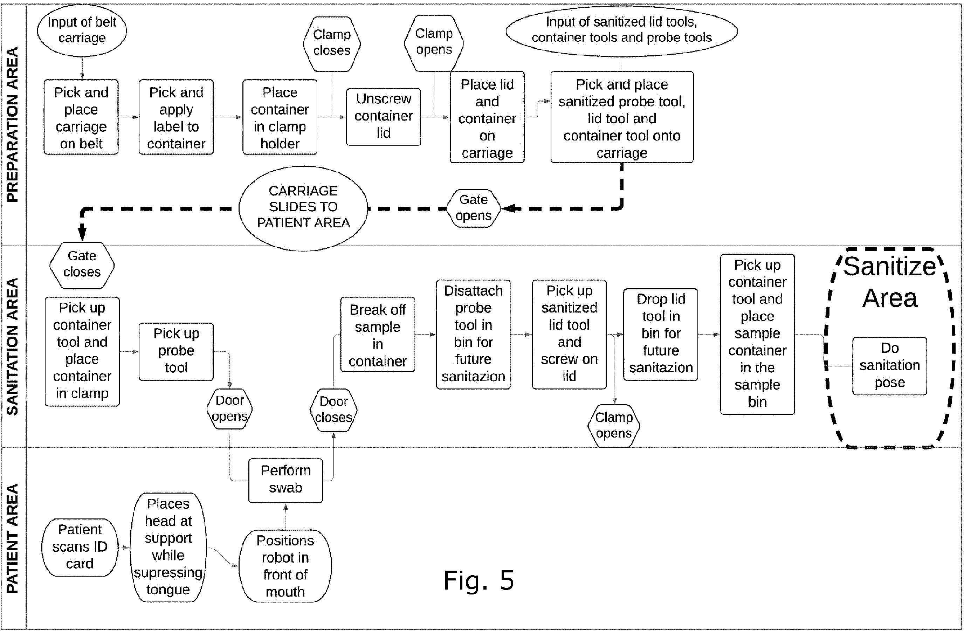

The patient area 3 is equipped with an adjustable headrest 16 to ensure a stable and comfortable position for the patient. Through testing it is only found necessary with the chin support. This is considered advantageous since it allows for the patient to retract their head at any time. A feature embedding a tongue depressor may also be provided in the apparatus. The patient area doors 15 are only open when the swap operation is executed and closed immediately after while handling the sample before finishing the cycle in the flow chart of fig. 5.

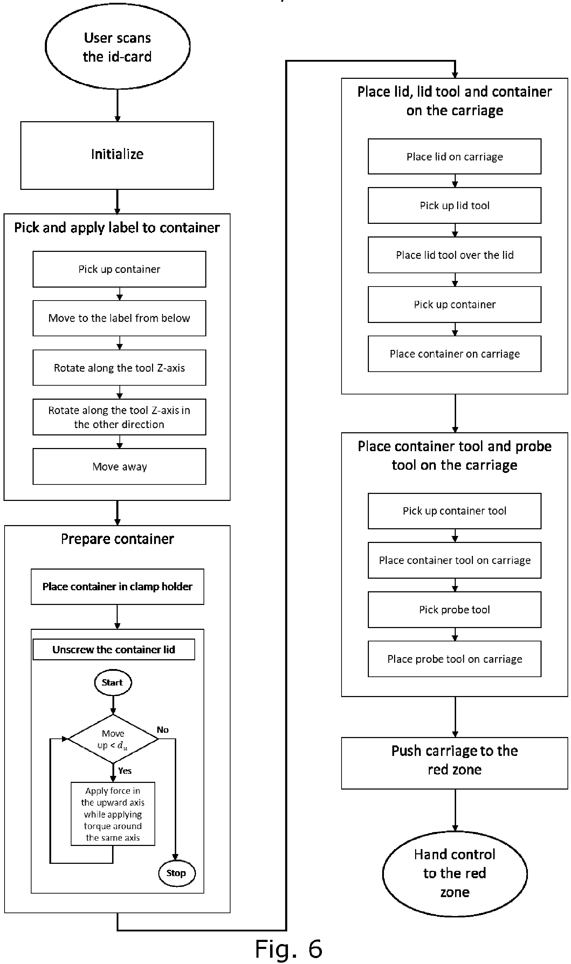

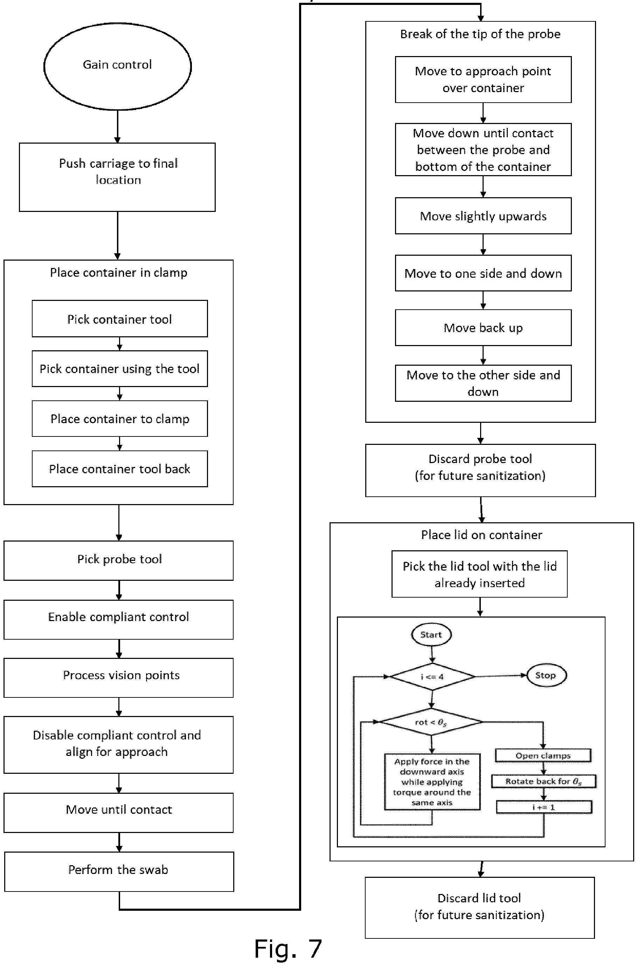

An overview of the software controlling the operations is illustrated in the flowcharts of fig. 6 and fig. 7. In fig. 6 there is shown the first robot 5 and periphery control in the preparation compartment (the green area), i.e. the section of the sampling apparatus that is separated from the patient. Control of the second robot 14 and periphery elements in the sanitation compartment (the red zone) is presented in fig. 7, which is further divided with additional information on specific parts of control.

As explained above, the green area is the section of the robotic sampling apparatus according to the invention not in contact with the patient. It comprises its own robot with a gripper attached. As shown in figures 5 and 6, the process is started by the user scanning their id-card. This triggers the initialization step which consists of setting up certain parameters and moving the first robot 5 to a safe initial (joint) configuration. As the card scan also triggers the printing of the label, the first robot 5 can now pick up the container and apply the label to it. The container is picked up such that the end-effector's z-axis is co-aligned with the major axis of the container. To ensure the label is applied properly, the robot 5 rotates the container in both directions along its major axis, while pressing upwards against a support.

In the next step, the container is opened. This is done by placing the container into the clamp the proper holder, which firmly holds it in place. The first robot 5, which holds the container by the lid, then opens the container. The first robot 5 applies forces upward along the major axis of the container while applying torque around the same axis. Once, due to compliance in the force controller, the robot has moved from the starting lid position, and the lid is fully unscrewed and move to the next step.

A carriage is used to transfer objects from the green area to the red area. The unscrewed lid is put on the carriage first. The first robot 5 then picks up the lid tool and pushes it over the placed lid. This will enable the second robot 14 to later pick it up. Next, the previously opened lid-tool, container is placed on the carriage. The tools, which the second robot 14 will need, are placed on the late to carriage next. In this regard, it is found that the orientation of the tools is important.

After all the needed tools and materials are on the carriage, the first robot 5 pushes the carriage to the red zone. As the used robot may have a limited workspace, the push is done in two steps. After this final step, the control is handed to the red zone, and the first robot is not used anymore in this run.

In the red area (the sanitation compartment and the patient area) the swab is performed. This is the only area of the sampling apparatus where the user is present. Similar to the green area, it comprises the second robot and peripheries. The second robot in this area is not equipped with a gripper, but rather a pneumatically activated tool exchange system. The process diagram for this area is presented in fig. 7.

After this area gains control over the procedure, it pushes the carriage to its final configuration, in which all the tools and equipment can be reached by the second robot. To firmly secure the container, this area also includes a pneumatically activated container clamp. The second robot picks the container tool from the carriage rack, picks the container, and places it in the clamp which is then activated. Before proceeding, the container tool is placed back on the carriage rack.

The next big step starts by picking up the probe tool, as it includes taking the sample. It then moves the second robot to an initial position and enables robot's compliance. The user can then freely move the second robot using the handles mounted at the end-effector. When the user positions the second robot so that the back of his or her throat is visible by the camera, the control is handed back to the second robot. The next step receives inputs from a vision part so they can be used in the swab procedure.

As the next steps are performed in close proximity to the user, they are performed with realtime control. The second robot moves the probe into the user's mouth until it touches the throat. This step can be roughly divided into two parts: moving until contact and performing the swab.

After the swab is performed, the robot breaks off the tip of the probe into the container, which is firmly positioned in the clamp. This is done in several steps: 1) the robot moves the probe pointing downward above the container, 2) it moves the probe until contact with the bottom of the container is detected, 3) it moves the probe slightly upwards, 4) then to one side and down, 5) back up, and 6) to the other side and down again. After this step breaks the tip of the probe, the probe tool is discarded or moved to the bin 19 for possible future sanitation.

In the last step the lid is placed back on the container. The robot attaches the lid tool with the inserted lid. The container is then closed by screwing on the lid. As the first robot has the camera cable and air supply connected to the tool, limited rotation range is available at the last axis. Because of this limitation the screwing is done in multiple steps. At each step it rotates the lid angularly while applying downward force along the major axis of the container. After each rotation, the clamps are opened and the robot rotates in the opposite direction, thus turning the whole container. After the step is repeated four times, the lid is fully attached, and the robot moves up while the clamps are closed. The empty lid tool can then be discarded or placed into the bin 19 for further sanitation.

The entire swab taking process is illustrated in figure 5 where there is shown a task flow. This task flow is organised as a "one-way-flow", describing the tool flow from one compartment to the next without backtracking. The task flow is determined based on the requirements for obtaining a high-yield throat swab sample while safely handling the sample with the objective of minimizing the risks of human infection. In addition, the task of equipment sanitation, which has to be executed after every sample is taken, is also addressed.

All of the operations in the preparation compartment 1 are executed with the robot 5. In some embodiments, in addition the operations in this area can also be executed by a human operator.

Although the present invention has been described in connection with the specified embodiments, it should not be construed as being in any way limited to the presented examples. The scope of the present invention is set out by the accompanying claim set. In the context of the claims, the terms "comprising", "comprises", "including" or "includes" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A robotic sampling apparatus for obtaining a biological sample from a patient, comprising
- a preparation compartment comprising
a plurality of storage areas accommodating individual test kit objects and a transfer arrangement for accommodating and repositioning at least one test kit object, and
a first handling system for collecting at least one test kit object from at least one of said storage areas and positioning said at least one test kit object in said transfer arrangement;
- a sanitation compartment, which is adapted to receiving the at least one test kit object on the transfer arrangement, and wherein said sanitation compartment comprises
a second handling system for picking a test kit object for obtaining the biological sample from the patient,
an opening for a patient area for providing the patient in a predetermined position which is accessible to the second handling system for moving said test kit object from an initial position to an active position in which the biological sample can be obtained from the patient; and
- a control system provided for governing at least the first handling system, the movement of the transfer arrangement and the second handling system for performing of the robotic sampling apparatus when obtaining the biological sample from a patient.

2. An apparatus according to claim 1, wherein the preparation compartment is environmentally controlled and preferably a sterile environment is kept therein.

3. An apparatus according to claim 1 or 2, wherein the test kit objects include a sample container with a lid, a sample probe tool with a sample probe and a carriage adapted for accommodating a container with a lid and a probe tool.

4. An apparatus according to any of the preceding claims, wherein the storage areas include exchangeable cartridges for each of the test kit objects.

5. An apparatus according to any of the preceding claims, wherein the preparation compartment further comprises a labelling device for printing a personalised label based on identification data from the patient and that the label is applied to the container on the transfer arrangement.

6. An apparatus according to any of the preceding claims, wherein the preparation compartment and the sanitation compartment are divided by an isolated seal, such as a dividing wall with an automated gate therein.

7. An apparatus according to claim 1, wherein the transfer arrangement is adapted to moving the at least one test kit object thereon from the preparation compartment to the sanitation compartment through the automated gate.

8. An apparatus according to any of the preceding claims, wherein the transfer arrangement is provided as a conveyor belt on which the at least one test kit object is placed in the preparation compartment and then transferred to the sanitation compartment.

9. An apparatus according to any of the preceding claims, wherein the sanitation compartment comprises an outlet for receiving the obtained sample herein for further processing, and a disposal bin for collecting objects other than the sample probe used for obtaining the biological sample.

10. An apparatus according to any of the preceding claims, wherein the sanitation compartment comprises a pneumatically actuated clamp for fixing a container for screwing on the lid by the second handling system.

11. An apparatus according to any of the preceding claims, wherein one or more the sterilisation systems are provided for decontamination of at least the sanitation compartment and the transfer arrangement.

12. An apparatus according to any of the preceding claims, wherein the sterilisation systems may include ultraviolet radiation, application of disinfectants or a combination thereof.

13. An apparatus according to any of the preceding claims, wherein the biological sample from the patient is a throat swab sample and the predetermined position of the patient in the patient area comprises a head support for providing the mouth of the patient in an accessible manner to the second handling system.

14. An apparatus according to claim 5, wherein the patient area further comprises a data input device, such as a card reader, for receiving identification data from the patient and obtaining label data to the labelling device, said label data may comprise the identification data and/or database data associated with said identification data.

15. An apparatus according to any of the preceding claims, wherein the first handling system is a robot and/or the second handling system is a robot.

16. A method of obtaining a biological sample from a patient, said method comprising
- preparing a sample probe for testing in a preparation compartment comprising a plurality of storage areas accommodating individual test kit objects and a transfer arrangement, and a first handling system for collecting at least one test kit object from at least one of said storage areas and positioning said at least one test kit object in said transfer arrangement;
- receiving the at least one test kit object on the transport device in a sanitation compartment, and wherein said sanitation compartment comprises a second handling system, an opening for a patient area for providing the patient in a predetermined position which is accessible to the second handling system, and
- moving said test kit object with the second handling system from an initial position to an active position where the biological sample is obtained from the patient by performing a swab,
- dispatching the obtained sample in an outlet for further processing, and collecting objects other than the sample probe used for obtaining the biological sample in a disposal bin; and
- controlling in a control system at least the first handling system, the movement of the transfer arrangement and the second handling system for operating a robotic sampling apparatus according to any of the preceding claims for obtaining the biological sample from a patient.

17. A method according to claim 16, whereby after having obtained the biological sample, the method further comprises the step of sterilising at least the sanitation compartment and the transfer arrangement.
